(19) Europäisches Patentamt
European Patent Office
Office européen des brevets

(11) Publication number: **0 322 892 B1**

(12) **EUROPEAN PATENT SPECIFICATION**

(45) Date of publication of patent specification: **25.03.92**

(51) Int. Cl.[5]: **A61K 31/495**, A61K 31/47, A61K 31/535, A61K 9/14, A61K 47/00

(21) Application number: **88121790.5**

(22) Date of filing: **28.12.88**

# (54) Freeze-dried pharmaceutical preparations for parenteral use.

(30) Priority: **28.12.87 JP 334923/87**

(43) Date of publication of application:
**05.07.89 Bulletin 89/27**

(45) Publication of the grant of the patent:
**25.03.92 Bulletin 92/13**

(84) Designated Contracting States:
**BE CH DE ES FR GB IT LI NL SE**

(56) References cited:
**EP-A- 0 067 666**
**EP-A- 0 138 018**
**EP-A- 0 187 315**
**EP-A- 0 195 316**
**EP-A- 0 219 784**

**CHEMICAL ABSTRACTS, vol. 106, no. 1, 5th January 1987, pages 470-471, abstract no. 4996j, Columbus, Ohio, US; & JP-A-61 137 885 (DAINIPPON PHARMACEUTICAL CO., LTD) 25-06-1986**

(73) Proprietor: **KYORIN SEIYAKU KABUSHIKI KAISHA**
**2-5, Kanda Surugadai**
**Chiyoda-ku Tokyo(JP)**

(72) Inventor: **Kamijo, Shinji**
**5-38-10, Soshigaya**
**Setagaya-ku Tokyo(JP)**
Inventor: **Imai, Jun**
**1400-70, Kosugaya-cho Sakae-ku**
**Yokohama-shi Kanagawa-ken(JP)**
Inventor: **Kodaira, Hiromichi**
**4660-4, Tomonuma Nogi-cho**
**Shimotsuga-gun Tochigi-ken(JP)**

(74) Representative: **Vossius & Partner**
**Siebertstrasse 4 P.O. Box 86 07 67**
**W-8000 München 86(DE)**

**EP 0 322 892 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to freeze-dried pharmaceutical preparations containing antibacterial quinolinecarboxylic acid derivatives which are characterized by their excellent stability against light and heat and are readily soluble.

In this invention, the objective compounds have the following general formula (I),

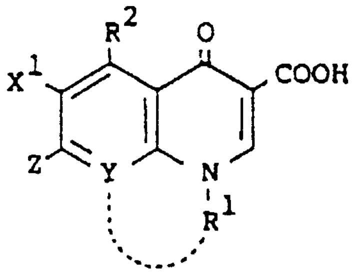

(I)

wherein $R^1$ represents a cycloalkyl group having 3 to 6 carbon atoms, straight or branched lower (hereinafter "lower" means 1-3 carbon atoms) alkyl group, haloalkyl group, alkenyl group, hydroxyalkyl group, lower alkylamino group or phenyl group which may be substituted, $R^2$ represents a hydrogen atom, halogen atom, nitro group or amino group, $X^1$ represents a halogen atom, Y represents a $=CX^2$- (wherein $X^2$ represents a halogen atom, alkyl group or alkoxy group, or $X^2$ and $R^1$ work together to be $-O-CH_2CH(CH_3)-$, $-CH_2CH_2CH(CH_3)-$ or $-CH_2CH(CH_3)-$), Z represents

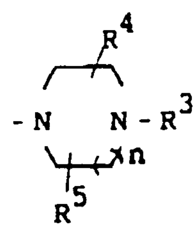

(wherein n is 1 or 2, $R^3$ represents a hydrogen atom or lower alkyl group, $R^4$ and $R^5$ each represent independently a hydrogen atom, lower alkyl group, aminoalkyl group, hydroxyalkyl group or phenyl group) or

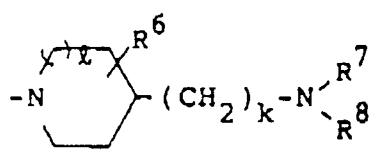

(wherein k is 0 or 1, ℓ is 0, 1 or 2, $R^6$ represents a hydrogen atom, halogen atom, lower alkyl group or hydroxy group, $R^7$ represents a hydrogen atom, lower alkyl group, haloalkyl group or hydroxyalkyl group, $R^8$ represents a hydrogen atom or lower alkyl group); the hydrates or the pharmaceutically acceptable acid addition or alkali salts thereof. It has been known that most of these compounds have relatively low solubility in water. There have been a variety of studies to improve the solubility. For instance, a solution of antibacterial quinolinecarboxylic acid derivatives could be prepared by adding lactic acid or the like (JP-A-60-94910 which corresponds to EP-A-0 138 018) or alkali (JP-A- 61-180771 which corresponds to EP-A-0 187 315). While, even after the dissolution of the compounds in water was achieved, it was difficult to develop a stable pharmaceutical solution proper for parenteral use, because the solution easily discolored under exposure to light or by heating and formed some decomposed materials. Therefore, a pharmaceutical solution containing antibacterial quinolinecarboxylic acid derivative proper for parenteral use has not been provided by the conventional manner.

The present inventors have extensively studied on stable pharmaceutical preparations containing antibacterial quinolinecarboxylic acid derivatives which are stable against light or heat and finally reached to

the present invention.

That is, the present inventors prepared, for the first time, freeze-dried pharmaceutical preparations containing antibacterial quinolinecarboxylic acid derivatives and found such freeze-dried pharmaceutical preparations are extremely stable against either light or heat and have good solubility in water for parenteral use. Thus, the preparations of the present invention have solved the problems hitherto known.

The freeze-dried pharmaceutical preparations of the present invention can be prepared as follows.

The quinolinecarboxylic acid derivative is dissolved by adding acid, alkali and/or water, immediately followed by refrigeration under -40 °C. It is then freeze-dried under a vacuous pressure using vacuum pump to give a fine cake.

The acid is preferably lactic acid, acetic acid or hydrochloric acid, and the alkali is preferably sodium hydroxide or potassium hydroxide. The concentration of quinolinecarboxylic acid derivative in the solution to be refrigerated may be within a range from 0.1 to 30 w/v%, preferably from 5 to 15 w/v%. If the concentration is less than 0.1 w/v%, the volume of the solution becomes to be large and a large vessel or a large injector will be required, which brings an inconvenience for use. On the other hand, if the concentration is higher than 30 w/v%, freeze-drying becomes difficult since the solution cannot be freezed easily by the refrigeration.

The solution may be sterilized by filtration through a membrane filter (pore size 0.22 $\mu$m) and then be filled into vials. The solution in vials is cooled to below -40 °C. As a general procedure, the vials are directly cooled by contacting with a refrigerant so as to obtain a rapid cooling. The cooling condition can be selected according to the ability of refrigerant and/or refrigerator, but no limitation is needed with respect to the cooling condition. Whatever condition to refrigerate below -40 °C is applicable.

The freeze-drying can be performed according to a general procedure under a vacuous pressure using vacuum pump and mild elevation of temperature. As for the freeze-drying method, the conventional heat shock method or a method characterized by adding a solvent can also be applied. After drying is completed, the vials are sealed, for example, with rubber stopper.

The freeze-dried pharmaceutical preparations of the present invention may be added with some medically usable adjuvants such as excipients, adjuvant to give the isotonicity, pH adjuster, stabilizer, solubilizer, buffering agents and preservatives. The excipients or the adjuvant to give the isotonicity may be xylitol, D-sorbitol, D-mannitol, fructose, glucose, sucrose, lactose, gelatin. The pH adjuster may also be lactic acid, acetic acid, hydrochloric acid, sodium hydroxide or potassium hydroxide.

The following examples illustrate the preferred embodiments of the present invention,

In the following examples, quinolinecarboxylic acid derivatives used are 6,8-difluoro-1-(2-fluoroethyl)-1,4-dihydro-7-(4-methyl-1-piperazinyl)-4-oxo-3-quinolinecarboxylic acid (hereinafter referred to as AM-833), 9-fluoro-2,3-dihydro-3-methyl-10-(4-methyl-1-piperazinyl)-7-oxo-7H-pyrido[1,2,3-de][1,4]-benzoxazine-6-carboxylic acid (hereinafter referred to as OFLX), 7-(3-amino-1-pyrrolidinyl)-8-chloro-1-cyclopropyl-6-fluoro-1,4-dihydro-4-oxo-3-quinolinecarboxylic acid (hereinafter referred to as AM-1091), 1-ethyl-6,8-difluoro-1,4-dihydro-7-(3-methyl-1-piperazinyl)-4-oxo-3-quinolinecarboxylic acid (hereinafter referred to as NY-198), 7-(3-amino-1-pyrrolidinyl)-1-(2,4-difluorophenyl)-6-fluoro-1,4-dihydro-4-oxo-1,8-naphthyridine-3-carboxylic acid p-toluenesulfonic acid hydrate (hereinafter referred to as T-3262).

Example 1

10 g of powdered AM-833 was dissolved in 50 ml of 1 M lactic acid, the pH was adjusted to 4.5 with 1 N sodium hydroxide solution, and filled with distilled water for injection to 100 ml. This solution was filtered through a membrane filter (pore size 0.22 $\mu$m) and each 2 ml of the filtrate was filled into the clean and sterilized vials. These vials were refrigerated by cooling to -42 °C, and dried under the vacuous pressure. The temperature of the shelf was kept at -20 °C during the initial stage (upto 22 hours) of drying. Under the vacuous pressure, the temperature was elevated to 20 °C and kept for 24 hours, and it was further elevated to 40 °C and kept for 6 hours to give the freeze-dried pharmaceutical preparation containing AM-833.

Example 2

15 g of powdered AM-833 was dissolved in 75 ml of 1 M lactic acid, pH was adjusted to 4.0 with 1 M sodium hydroxide solution, and filled with distilled water for injection to 100 ml. This solution was filtered through a membrane filter (pore size 0.22 $\mu$m), and each 1.33 ml of the filtrate was filled into the clean and sterilized vials. These vials were refrigerated by cooling to -40 °C, and dried under the vacuous pressure in a similar manner as example 1 to give the freeze-dried pharmaceutical preparation containing AM-833.

Example 3

10 g of powdered AM-833 and 10 g of glucose were dissolved in 50 ml of 1 M lactic acid, pH was adjusted to 4.5 with 1 M sodium hydroxide solution, and filled with distilled water for injection to 100 ml. This solution was filtered through a membrane filter (pore size 0.22 $\mu$m), and each 2 ml of the filtrate was filled into the clean and sterilized vials. These vials were refrigerated by cooling to -40 °C. The temperature was elevated to -10 °C, and kept for 5 hours, Then, it was cooled again to -40 °C and dried under the vacuous pressure. The temperature of the shelf was kept at -20 °C during the initial stage (upto 66 hours) of drying. Under the vacuous pressure, the temperature was elevated to 20 °C and kept for 5 hours to give the freeze-dried pharmaceutical preparation containing AM-833.

Example 4

10 g of powdered AM-833 was dissolved in 55 ml of 1 N sodium hydroxide solution, and the pH was adjusted to 10.1 with 1 M lactic acid, and filled with distilled water for injection to 100 ml. This solution was filtered through a membrane filter (pore size 0.22 $\mu$m), and each 2 ml of the filtrate was filled into the clean and sterilized vials. These vials were refrigerated by cooling to -44 °C and dried under the vacuous pressure. The temperature of the shelf was kept at -20 °C during the initial stage (upto 22 hours) of drying. Under the vacuous pressure, the temperature was elevated to 20 °C and kept for 26 hours, and it was further elevated to 40 °C and kept for 20 hours to give the freeze-dried pharmaceutical preparation containing AM-833.

Example 5

5 g of powdered OFLX was dissolved in 25 ml of 1 M lactic acid and filled with distilled water for injection to 100 ml. This solution was filtered through a membrane filter (pore size 0.22 $\mu$m), and each 2 ml of the filtrate was filled into the clean and sterilized vials. These vials were cooled to -40 °C and dried under the vacuous pressure in a similar manner as example 1 to give the freeze-dried pharmaceutical preparation containing OFLX.

Example 6

5 g of powdered AM-1091 was dissolved in distilled water for injection and made up to 100 ml with that. This solution was filtered through a membrane filter (pore size 0.22 $\mu$m), and each 2 ml of the filtrate was filled into the clean and sterilized vials. These vials were refrigerated by cooling to -40 °C and dried under the vacuous pressure. The temperature of the shelf was kept at -20 °C during the initial stage (upto 5 hours) of drying. Subsequently, the temperature was elevated to 20 °C and kept for 63 hours for drying under the vacuous pressure to give the freeze-dried pharmaceutical preparation containing AM-1091.

Example 7

5 g of powdered AM-1091 was dissolved in 25 ml of 1 N sodium hydroxide solution and filled with distilled water for injection to 100 ml. This solution was filtered through a membrane filter (pore size 0.22 $\mu$m), and each 2 ml of the filtrate was filled into the clean and sterilized vials. These vials were refrigerated by cooling to -40 °C. The temperature was elevated to -10°C and kept for 5 hours. Then, it was cooled again to -40 °C, and dried under the vacuous pressure. The temperature of the shelf was kept at -20 °C during the initial stage (upto 6 hours) of drying. Subsequently, the temperature was elevated to 20 °C and kept for 21 hours for drying under the vacuous pressure to give the freeze-dried pharmaceutical preparation containing AM-1091.

Example 8

1 g of powdered NY-198 was dissolved in 20 ml of acetic acid and filled with distilled water for injection to 50 ml. This solution was filtered through a membrane filter (pore size 0.22 $\mu$m), and each 2 ml of the filtrate was filled into the clean and sterilized vials. These vials were refrigerated by cooling to -40 °C and dried under the vacuous pressure in a similar manner as example 14 to give the freeze-dried pharmaceutical preparation containing NY-198.

Stability test

The freeze-dried pharmaceutical preparations obtained were evaluated by the stability test, under the light (fluorescence lamp, 1,200,000 Lux•hours) and heat (50 °C, 3 months) as compared with the solution prepared by using same components.

Results are shown in table 1. These freeze-dried pharmaceutical preparations of the present invention have excellent properties, particularly with respect to stability as compared with those of the solutions.

Table 1

| Result of the stability of the freeze-dried pharmaceutical preparations of this inventions and the solutions | | | | |
|---|---|---|---|---|
| Examples No. | light (fluorescence lamp) | | heat (50 °C, 3 months) | |
| | freeze-dried | solution | freeze-dried | solution |
| 1 | no changes | yellowish | no changes | yellowish |
| 5 | no changes | slightly yellowish | no changes | no changes |
| 6 | no changes | precipitate of light brown | no changes | no changes |
| 8 | no changes | slightly brown | no changes | no changes |

## Claims

**Claims for the following Contracting States : BE, CH, DE, FR, GB, IT, LI, NL, SE**

**1.** A freeze-dried pharmaceutical preparation containing the compound of the following formula (I),

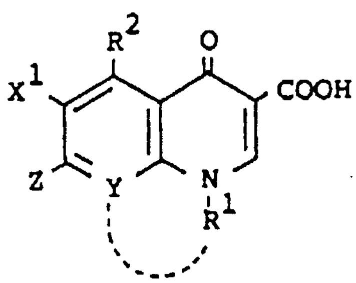

(I)

wherein $R^1$ represents a cycloalkyl group having 3 to 6 carbon atoms, straight or branched lower alkyl group, haloalkyl group, alkenyl group. hydroxyalkyl group, lower alkylamino group or phenyl group which may be substituted, $R^2$ represents a hydrogen atom, halogen atom, nitro group or amino group, $X^1$ represents a halogen atom, Y represents $=CX^2$- (wherein $X^2$ represents a halogen atom, alkyl group or alkoxy group, or $X^2$ and $R^1$ work together to be $-O-CH_2CH(CH_3)-$, $-CH_2CH_2CH(CH_3)-$ or $-CH_2CH(CH_3)-$), Z represents

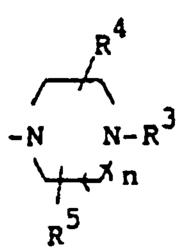

(wherein n is 1 or 2, $R^3$ represents a hydrogen atom or lower alkyl group, $R^4$ and $R^5$ each represent independently a hydrogen atom, lower alkyl group, aminoalkyl group, hydroxyalkyl group or phenyl group) or

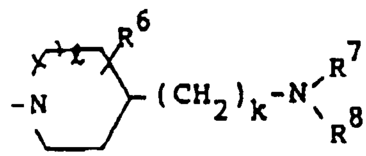

(wherein k is 0 or 1, ℓ is 0, 1 or 2, $R^6$ represents a hydrogen atom, halogen atom, lower alkyl group or hydroxy group, $R^7$ represents a hydrogen atom, lower alkyl group, haloalkyl group or hydroxyalkyl group, $R^8$ represents a hydrogen atom or lower alkyl group); the hydrates or the pharmaceutically acceptable acid addition or alkali salts thereof.

2. A freeze-dried pharmaceutical preparation as claimed in Claim 1, in which the compound is 6,8-difluoro-1-(2-fluoroethyl)-1,4-dihydro-7-(4-methyl-1-piperazinyl)-4-oxo-3-quinolinecarboxylic acid.

3. A freeze-dried pharmaceutical preparation as claimed in Claim 1, in which the compound is 9-fluoro-2,3-dihydro-3-methyl-10-(4-methyl-1-piperazinyl)-7-oxo-7H-pyrido[1,2,3-de][1,4]-benzoxazine-6-carboxylic acid.

4. A freeze-dried pharmaceutical preparation as claimed in Claim 1, in which the compound is 7-(3-amino-1-pyrrolidinyl)-8-chloro-1-cyclopropyl-6-fluoro-1,4-dihydro-4-oxo-3-quinolinecarboxylic acid.

5. A freeze-dried pharmaceutical preparation as claimed in Claim 1, in which the compound is 1-ethyl-6,8-difluoro-1,4-dihydro-7-(3-methyl-1-piperazinyl)-4-oxo-3-quinolinecarboxylic acid.

6. A method for preparing the freeze-dried pharmaceutical preparation as claimed in Claim 1, which comprises dissolving the above-mentioned compound in water containing acid or alkali, followed by freeze-drying the solution.

7. A method of claim 6, wherein the acid is hydrochloric acid, lactic acid or acetic acid.

8. A method of claim 6, wherein the alkali is sodium hydroxide or potassium hydroxide.

**Claims for the following Contracting State : ES**

1. A method for preparing a freeze-dried pharmaceutical preparation containing the compound of the following formula (I),

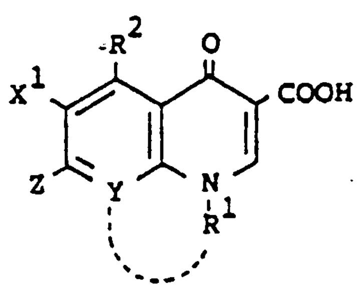

(I)

wherein $R^1$ represents a cycloalkyl group having 3 to 6 carbon atoms, straight or branched lower alkyl group, haloalkyl group, alkenyl group, hydroxyalkyl group, lower alkylamino group or phenyl group which may be substituted, $R^2$ represents a hydrogen atom, halogen atom, nitro group or amino group, $X^1$ represents a halogen atom, Y represents $=CX^2-$ (wherein $X^2$ represents a halogen atom, alkyl group or alkoxy group, or $X^2$ and $R^1$ work together to be $-O-CH_2CH(CH_3)-$, $-CH_2CH_2CH(CH_3)-$ or $-CH_2CH(CH_3)-$), Z represents

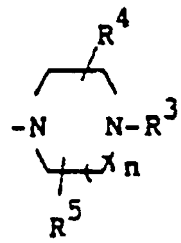

(wherein n is 1 or 2, $R^3$ represents a hydrogen atom or lower alkyl group, $R^4$ and $R^5$ each represent independently a hydrogen atom, lower alkyl group, aminoalkyl group, hydroxyalkyl group or phenyl group) or

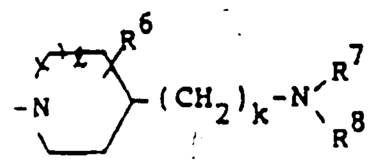

(wherein k is 0 or 1, ℓ is 0, 1 or 2, $R^6$ represents a hydrogen atom, halogen atom, lower alkyl group or hydroxy group, $R^7$ represents a hydrogen atom, lower alkyl group, haloalkyl group or hydroxyalkyl group, $R^8$ represents a hydrogen atom or lower alkyl group); the hydrates or the pharmaceutically acceptable acid addition or alkali salts thereof which comprises dissolving the above-mentioned compound or its hydrates or salts in water containing acid or alkali, followed by freeze-drying the solution.

**2.** A method of claim 1, wherein the acid is hydrochloric acid, lactic acid or acetic acid.

**3.** A method of claim 1, wherein the alkali is sodium hydroxide or potassium hydroxide.

**4.** The method according to any one of claims 1 to 3 wherein the compound used is 6,8-difluoro-1-(2-fluoroethyl)-1,4-dihydro-7-(4-methyl-1-piperazinyl)-4-oxo-3-quinolinecarboxylic acid.

**5.** The method according to any one of claims 1 to 3 wherein the compound used is 9-fluoro-2,3-dihydro-3-methyl-10-(4-methyl-1-piperazinyl)-7-oxo-7H-pyrido[1,2,3-de][1,4]-benzoxazine-6-carboxylic acid.

**6.** The method according to any one of claims 1 to 3 wherein the compound used is 7-(3-amino-1-pyrrolidinyl)-8-chloro-1-cyclopropyl-6-fluoro-1,4-dihydro-4-oxo-3-quinolinecarboxylic acid.

**7.** The method according to any one of claims 1 to 3 wherein the compound used is 1-ethyl-6,8-difluoro-1,4-dihydro-7-(3-methyl-1-piperazinyl)-4-oxo-3-quinolinecarboxylic acid.

**Revendications**

**Revendications pour les Etats contractants suivants : BE, CH, DE, FR, GB, IT, LI, NL, SE**

**1.** Préparation pharmaceutique lyophilisée contenant le composé de formule (I) suivante :

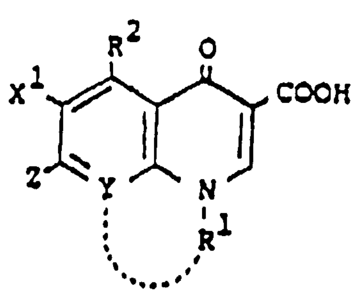

(I)

dans laquelle $R^1$ représente un groupe cycloalkyle ayant 3 à 6 atomes de carbone, un groupe alkyle inférieur linéaire ou ramifié, un groupe haloalkyle, un groupe alcényle, un groupe hydroxyalkyle, un groupe alkylamino inférieur ou un groupe phényle qui peut être substitué, $R^2$ représente un atome d'hydrogène, un atome d'halogène, un groupe nitro ou un groupe amino, $X^1$ représente un atome d'halogène, Y représente $=CX^2$- (dans lequel $X^2$ représente un atome d'halogène, un groupe alkyle ou un groupe alkoxy, ou bien $X^2$ et $R^1$ conjointement constituent le groupe $-O-CH_2CH(CH_3)-$, $-CH_2CH_2CH(CH_3)-$ ou $-CH_2CH(CH_3)-$), Z représente :

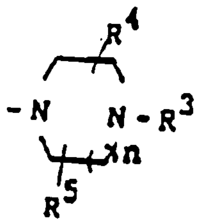

(dans laquelle formule n est 1 ou 2, $R^3$ représente un atome d'hydrogène ou un groupe alkyle inférieur, $R^4$ et $R^5$ représentent chacun indépendamment un atome d'hydrogène, un groupe alkyle inférieur, un groupe alkylamino, un groupe hydroxyalkyle ou un groupe phényle) ou :

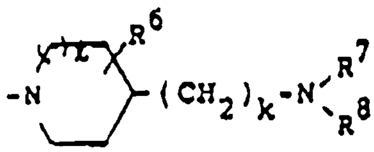

(dans laquelle formule k est 0 ou à 1, ℓ est 0, 1 ou 2, $R^6$ représente un atome d'hydrogène, un atome d'halogène, un groupe alkyle inférieur ou un groupe hydroxy, $R^7$ représente un atome d'hydrogène, un groupe alkyle inférieur, un groupe haloalkyle ou un groupe hydroxyalkyle, $R^8$ représente un atome d'hydrogène ou un groupe alkyle inférieur); les hydrates ou les sels d'addition acides ou les sels alcalins pharmaceutiquement acceptables de ces composés.

**2.** Préparation pharmaceutique lyophilisée selon la revendication 1, dans laquelle le composé est l'acide 6,8-difluoro-1-(2-fluoréthyl)-1,4-dihydro-7-(4-méthyl-1-pipérazinyl)-4-oxo-3-quinoléine carboxylique.

**3.** Préparation pharmaceutique lyophilisée selon la revendication 1, dans laquelle le composé est l'acide 9-fluoro-2,3-dihydro-3-méthyl-10-(4-méthyl-1-pipérazinyl)-7-oxo-7H-pyrido [1,2,3-de][1,4]-benzoxazine-6-carboxylique.

**4.** Préparation pharmaceutique lyophilisée selon la revendication 1, dans laquelle le composé est l'acide 7-(3-amino-1-pyrrolidinyl)-8-chloro-1-cyclopropyl-6- fluoro-1,4-dihydro-4-oxo-3-quinoléine carboxylique.

**5.** Préparation pharmaceutique lyophilisée selon la revendication 1, dans laquelle le composé est l'acide 1-éthyl-6,8-difluoro-1,4-dihydro-7-(3-méthyl-1-pipérazinyl)-4-oxo-3-quinoléine carboxylique.

**6.** Procédé de préparation de la préparation pharmaceutique lyophilisée selon la revendication 1, qui consiste à dissoudre le composé précité dans de l'eau contenant un acide ou un alcali, l'opération étant suivie d'une lyophilisation de la solution.

**7.** Procédé selon la revendication 6, dans lequel l'acide est l'acide chlorhydrique, l'acide lactique ou l'acide acétique.

**8.** Procédé selon la revendication 6, dans lequel l'alcali est l'hydroxyde de sodium ou l'hydroxyde de potassium.

**Revendications pour l'Etat contractant suivant : ES**

1. Procédé d'une préparation pharmaceutique lyophilisée contenant le composé de formule (I) suivante :

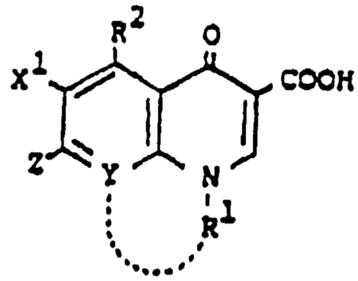

(I)

dans laquelle $R^1$ représente un groupe cycloalkyle ayant 3 à 6 atomes de carbone, un groupe alkyle inférieur linéaire ou ramifié, un groupe haloalkyle, un groupe alcényle, un groupe hydroxyalkyle, un groupe alkylamino inférieur ou un groupe phényle qui peut être substitué, $R^2$ représente un atome d'hydrogène, un atome d'halogène, un groupe nitro ou un groupe amino, $X^1$ représente un atome d'halogène, Y représente $=CX^2$- (dans lequel $X^2$ représente un atome d'halogène, un groupe alkyle ou un groupe alkoxy, ou bien $X^2$ et $R^1$ conjointement constituent le groupe $-O-CH_2CH(CH_3)-$, $-CH_2CH_2CH(CH_3)-$ ou $-CH_2CH(CH_3)-$), Z représente :

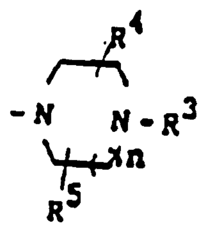

(dans laquelle formule n est 1 ou 2, $R^3$ représente un atome d'hydrogène ou un groupe alkyle inférieur, $R^4$ et $R^5$ représentent chacun indépendamment un atome d'hydrogène, un groupe alkyle inférieur, un groupe alkylamino, un groupe hydroxyalkyle ou un groupe phényle) ou :

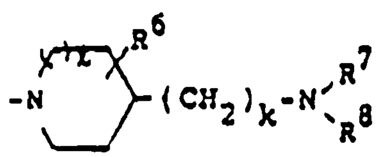

(dans laquelle formule k est 0 ou à 1, $\ell$ est 0, 1 ou 2, $R^6$ représente un atome d'hydrogène, un atome d'halogène, un groupe alkyle inférieur ou un groupe hydroxy, $R^7$ représente un atome d'hydrogène, un groupe alkyle inférieur, un groupe haloalkyle ou un groupe hydroxyalkyle, $R^8$ représente un atome d'hydrogène ou un groupe alkyle inférieur); les hydrates ou les sels d'addition acides ou les sels alcalins pharmaceutiquement acceptables de ces composés qui consiste à dissoudre le composé précité ou leurs hydrates et leurs sels dans de l'eau contenant un acide ou un alcali, l'opération étant suivie d'une lyophilisation de la solution.

2. Procédé selon la revendication 1, dans lequel l'acide est l'acide chlorhydrique, l'acide lactique ou l'acide acétique.

3. Procédé selon la revendication 1, dans lequel l'alcali est l'hydroxyde de sodium ou l'hydroxyde de potassium.

4. Préparation selon l'une quelconque des revendications 1 à 3, dans laquelle le composé utilisé est l'acide 6,8-difluoro-1-(2-fluoréthyl)-1,4-dihydro-7-(4-méthyl-1-pipérazinyl)-4-oxo-3-quinoléine carboxylique.

5. Préparation selon l'une quelconque des revendications 1 à 3, dans laquelle le composé utilisé est l'acide 9-fluoro-2,3-dihydro-3-méthyl-10-(4-méthyl-1-pipérazinyl)-7-oxo-7H-pyrido [1,2,3-de][1,4] benzoxazine-6-carboxylique.

6. Préparation selon l'une quelconque des revendications 1 à 3, dans laquelle le composé utilisé est l'acide 7-(3-amino-1-pyrrolidinyl)-8-chloro-1-cyclopropyl-6- fluoro-1,4-dihydro-4-oxo-3-quinoléine carboxylique.

7. Préparation selon l'une quelconque des revendications 1 à 3, dans laquelle le composé utilisé est l'acide 1-éthyl-6,8-difluoro-1,4-dihydro-7-(3-méthyl-1-pipérazinyl)-4-oxo-3-quinoléine carboxylique.

**Patentansprüche**

**Patentansprüche für folgende Vertragsstaaten : BE, CH, DE, FR, GB, IT, LI, NL, SE**

1. Gefriergetrocknetes Arzneimittel, enthaltend die Verbindung der folgenden Formel (I)

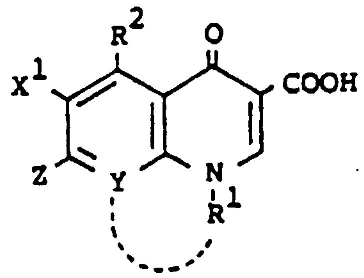

(I)

in der $R^1$ einen Cycloalkylrest mit 3 bis 6 Kohlenstoffatomen, einen geradkettigen oder verzweigten Niederalkylrest, einen Haloalkylrest, einen Alkenylrest, einen Hydroxyalkylrest, einen Niederalkylaminorest oder eine gegebenenfalls substituierte Phenylgruppe darstellt, $R^2$ ein Wasserstoffatom, ein Halogenatom, eine Nitrogruppe oder eine Aminogruppe bedeutet, $X^1$ ein Halogenatom darstellt, Y den Rest $=CX^2$- bedeutet (wobei $X^2$ ein Halogenatom, einen Alkylrest oder einen Alkoxyrest darstellt, oder $X_2$ und $R^1$ zusammen die Gruppe $-O-CH_2CH(CH_3)-$, $-CH_2CH_2CH(CH_3)-$ oder $-CH_2CH(CH_3)-$ bilden), Z den Rest

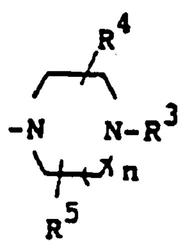

darstellt (wobei n den Wert 1 oder 2 hat, $R^3$ ein Wasserstoffatom oder einen Niederalkylrest darstellt, $R^4$ und $R^5$ jeweils unabhängig voneinander ein Wasserstoffatom, einen Niederalkylrest, einen Aminoalkylrest, einen Hydroxyalkylrest oder eine Phenylgruppe bedeuten) oder den Rest

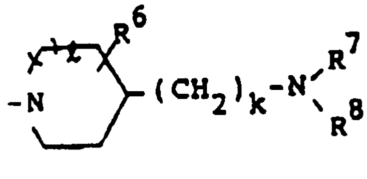

darstellt (wobei k den Wert 0 oder 1 hat, 1 den Wert 0, 1 oder 2 hat, $R^6$ ein Wasserstoffatom, ein Halogenatom, einen Niederalkylrest oder eine Hydroxylgruppe bedeutet, $R^7$ ein Wasserstoffatom, einen Niederalkylrest, einen Haloalkylrest oder einen Hydroxyalkylrest darstellt, $R^8$ ein Wasserstoffatom oder einen Niederalkylrest bedeutet); die Hydrate oder die pharmazeutisch verträglichen Säureadditions-

oder Alkalisalze davon.

2. Gefriergetrocknetes Arzneimittel nach Anspruch 1, in der die Verbindung 6,8-Difluor-1-(2-fluorethyl)-1,4-dihydro-7-(4-methyl-1-piperazinyl)-4-oxo-3-chinolincarbonsäure ist.

3. Gefriergetrocknetes Arzneimittel nach Anspruch 1, wobei die Verbindung 9-Fluor-2,3-dihydro-3-methyl-10-(4-methyl-1-piperazinyl)-7-oxo-7H-pyrido[1,2,3-de][1,4]-benzoxazin-6-carbonsäureist.

4. Gefriergetrocknetes Arzneimittel nach Anspruch 1, wobei die Verbindung 7-(3-Amino-1-pyrrolidinyl)-8-chloro-1-cyclopropyl-6-fluor-1,4-dihydro-4-oxo-3-chinolincarbonsäure ist.

5. Gefriergetrocknetes Arzneimittel nach Anspruch 1, wobei die Verbindung 1-Ethyl-6,8-difluor-1,4-dihydro-7-(3-methyl-1-piperazinyl)-4-oxo-3-chinolincarbonsäure ist.

6. Verfahren zur Herstellung des gefriergetrockneten Arzneimittels nach Anspruch 1, umfassend das Auflösen der vorstehend genannten Verbindung in Wasser, das eine Säure oder Base enthält, gefolgt von der Gefriertrocknung der Lösung.

7. Verfahren nach Anspruch 6, wobei die Säure Salzsäure, Milchsäure oder Essigsäure ist.

8. Verfahren nach Anspruch 6, wobei die Base Natriumhydroxid oder Kaliumhydroxid ist.

**Patentansprüche für folgenden Vertragsstaat : ES**

1. Verfahren zur Herstellung eines gefriergetrockneten Arzneimittels, enthaltend die Verbindung der folgenden Formel (I)

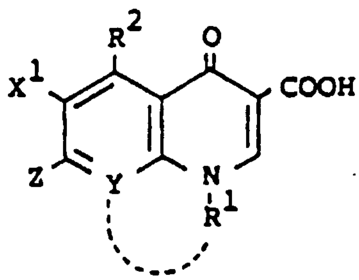

(I)

in der $R^1$ einen Cycloalkylrest mit 3 bis 6 Kohlenstoffatomen, einen geradkettigen oder verzweigten Niederalkylrest, einen Haloalkylrest, einen Alkenylrest, einen Hydroxyalkylrest, einen Niederalkylaminorest oder eine gegebenenfalls substituierte Phenylgruppe darstellt, $R^2$ ein Wasserstoffatom, ein Halogenatom, eine Nitrogruppe oder eine Aminogruppe bedeutet, $X^1$ ein Halogenatom darstellt, Y den Rest $=CX^2$- bedeutet (wobei $X^2$ ein Halogenatom, einen Alkylrest oder einen Alkoxyrest darstellt, oder $X_2$ und $R^1$ zusammen die Gruppe $-O-CH_2CH(CH_3)-$, $-CH_2CH_2CH(CH_3)-$ oder $-CH_2CH(CH_3)-$ bilden), Z den Rest

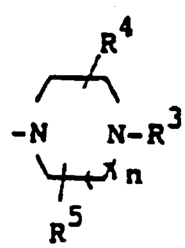

darstellt (wobei n den Wert 1 oder 2 hat, $R^3$ ein Wasserstoffatom oder einen Niederalkylrest darstellt, $R^4$ und $R^5$ jeweils unabhängig voneinander ein Wasserstoffatom, einen Niederalkylrest, einen Aminoalkylrest, einen Hydroxyalkylrest oder eine Phenylgruppe bedeuten) oder den Rest

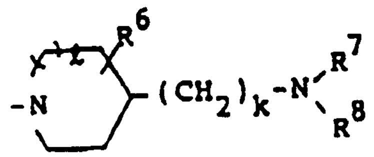

darstellt (wobei k den Wert 0 oder 1 hat, 1 den Wert 0, 1 oder 2 hat, $R^6$ ein Wasserstoffatom, ein Halogenatom, einen Niederalkylrest oder eine Hydroxylgruppe bedeutet, $R^7$ ein Wasserstoffatom, einen Niederalkylrest, einen Haloalkylrest oder einen Hydroxyalkylrest darstellt, $R^8$ ein Wasserstoffatom oder einen Niederalkylrest bedeutet); die Hydrate oder die pharmazeutisch verträglichen Säureadditions- oder Alkalisalze davon, umfassend das Lösen der vorstehend genannten Verbindung oder ihrer Hydrate oder Salze in Wasser, das eine Säure oder Base enthält, gefolgt von der Gefriertrocknung der Lösung.

2. Verfahren nach Anspruch 1, wobei die Säure Salzsäure, Milchsäure oder Essigsäure ist.

3. Verfahren nach Anspruch 1, wobei die Base Natriumhydroxid oder Kaliumhydroxid ist.

4. Verfahren nach einem der Ansprüche 1 bis 3, wobei die verwendete Verbindung 6,8-Difluor-1-(2-fluorethyl)-1,4-dihydro-7-(4-methyl-1-piperazinyl)-4-oxo-3-chinolincarbonsäure ist.

5. Verfahren nach einem der Ansprüche 1 bis 3, wobei die verwendete Verbindung 9-Fluor-2,3-dihydro-3-methyl-10-(4-methyl-1-piperazinyl)-7-oxo-7H-pyrido[1,2,3-de][1,4]-benzoxazin-6-carbonsäure ist.

6. Verfahren nach einem der Ansprüche 1 bis 3, wobei die verwendete Verbindung 7-(3-Amino-1-pyrrolidinyl)-8-chlor-1-cyclopropyl-6-fluor-1,4-dihydro-4-oxo-3-chinolincarbonsäure ist.

7. Verfahren nach einem der Ansprüche 1 bis 3, wobei die verwendete Verbindung 1-Ethyl-6,8-difluor-1,4-dihydro-7-(3-methyl-1-piperazinyl)-4-oxo-3-chinolincarbonsäure ist.